**Europäisches Patentamt**

**European Patent Office**

**Office européen des brevets**

(19)

(11) Veröffentlichungsnummer : **0 414 006 B1**

(12)
# EUROPÄISCHE PATENTSCHRIFT

(45) Veröffentlichungstag der Patentschrift :
06.10.93 Patentblatt 93/40

(51) Int. Cl.$^5$ : **A61M 1/34**

(21) Anmeldenummer : **90114842.9**

(22) Anmeldetag : **02.08.90**

(54) **Vorrichtung zur selektiven Elimination von Plasmabestandteilen aus Blut.**

(30) Priorität : **22.08.89 DE 3927633**

(43) Veröffentlichungstag der Anmeldung :
**27.02.91 Patentblatt 91/09**

(45) Bekanntmachung des Hinweises auf die
Patenterteilung :
**06.10.93 Patentblatt 93/40**

(84) Benannte Vertragsstaaten :
**DE ES FR GB IT**

(56) Entgegenhaltungen :
**EP-A- 0 232 884**
**DE-C- 3 245 591**
**US-A- 4 191 182**

(73) Patentinhaber : **Fresenius AG**
**Gluckensteinweg 5**
**D-61350 Bad Homburg (DE)**

(72) Erfinder : **Flaig, Hans-Jürgen, Dr.**
**Kantstrasse 11**
**D-6420 Lauterbach (DE)**
Erfinder : **von Baeyer, Hans, Prof.Dr.**
**Spandauer Damm 130**
**D-1000 Berlin 90 (DE)**

(74) Vertreter : **Dr. Fuchs, Dr. Luderschmidt Dr.**
**Mehler, Dipl.-Ing. Weiss Patentanwälte**
**Postfach 46 60, Abraham-Lincoln-Strasse 7**
**D-65036 Wiesbaden (DE)**

EP 0 414 006 B1

EP 0 414 006 B1

## Beschreibung

Die Erfindung betrifft eine Vorrichtung zur selektiven Elimination von Plasmabestandteilen aus Blut, wobei bei dem Verfahren mittels eines Separationsfilters Plasma vom Blut getrennt wird und aus dem Plasma anschliessend vor dessen Rückmischung mit den korpuskularen Blutbestandteilen mittels einer Abtrennvorrichtung die jeweiligen Plasmabestandteile entfernt werden. Die Erfindung bezieht sich auf eine Anordnung mit einer Blutzuführeinrichtung, einem Separationsfilter zur Abtrennung von Plasma von dem Blut, einer Abtrennvorrichtung zur Entfernung der jeweiligen Plasmabestandteile, einer Mischeinrichtung zum Mischen des prozessierten Plasmas mit den korpuskulären Bestandteilen des Blutes und einer Blutrückführeinrichtung.

Bei der Hämopherese werden Blutbestandteile unter Verwendung eines extrakorporalen Kreislaufs eliminiert. Die hierzu verwendeten Vorrichtungen umfassen üblicherweise einen Separationsfilter oder Plasmaseparator, mit Hilfe dessen das Plasma des Patientenblutes von den korpuskularen Blutbestandteilen getrennt wird. Während die korpuskularen Bestandteile im wesentlichen unbehandelt durch die Vorrichtung geleitet werden, ist es möglich, das Blutplasma durch weitere Filter- oder Abtrennvorrichtungen zu leiten, um diesem bestimmte Blutbestandteile zu entziehen.

Die selektive und spezifische Elimination von Plasmabestandteilen läßt sich anhand der LDL-Apherese besonders gut darstellen. Bei der LDL-Apherese wird das Lipoprotein niedriger Dichte (LDL) aus dem Plasma entfernt. Die LDL-Apherese wird beispielsweise bei Patienten mit genetisch bedingter Hyperlipoproteinämie Typ IIa angewandt. Aus dem Stand der Technik sind hierfür verschiedene Verfahren bekannt, beispielsweise die Membran-Differentialfiltration, die Immunadsorption oder die Heparin-Präzipitation. Bei all diesen Verfahren wird jeweils in einer ersten Stufe das Plasma von den korpuskularen Blutbestandteilen getrennt, um das Blutplasma nachfolgend separat verarbeiten oder behandeln zu können. Die Trennung des Plasmas von dem Blut erfolgt bei den bekannten Verfahren mittels eines Plasmafilters oder einer Zentrifuge, in dem zweiten Kreislauf, welcher den Plasmakreislauf bildet, ist es möglich, Membran-Differentialfilter oder Immunadsorber zu verwenden.

Bei den bekannten Vorrichtungen erweist es sich als nachteilig, daß die bei der Behandlung des Plasmas zu erzielenden Eliminationswerte sowohl apparativ als auch verfahrenstechnisch beschränkt sind. Zum einen erweist es sich als nachteilig, daß bei Membranfiltern durch die Porenverstopfung eine Beschränkung der Aufnahmekapazität gegeben ist und bei Adsorbern ähnliche Probleme auftreten. Es ist zwar möglich, mittels einer Rückspülung der Membran die Filtrationsfähigkeit des Membranmoduls oder Membranfilters zu reaktivieren, diese Vorgehensweise erfordert jedoch einen höheren apparatetechnischen Aufwand und erhöht insgesamt die Behandlungszeit des Patienten. Gleiches gilt für eine Desorption, mittels welcher unter Verwendung eines Zweisäulenverfahrens die Adsorptionskapazität der Adsorbersäule wieder herstellbar ist.

Die verfahrensmäßige Beschränkung der Eliminationskapazität besteht darin, daß beispielsweise bei der LDL-Elimination das in der ersten Verfahrensstufe gewonnene Blutplasmavolumen für die weitere Behandlung in der zweiten Behandlungsstufe ausschlaggebend ist. In Abhängigkeit von dem Plasmavolumen ergeben sich natürlichermaßen Grenzwerte für die Elimination der Plasmabestandteile.

Aus der DE-OS 32 45 591 ist es bekannt, das zu behandelnde Plasma zu verdünnen, bevor dieses einer LDL-Abtrennvorrichtung zugeführt wird. Auch hierbei erweist es sich als nachteilig, daß das von dem Blut des Patienten getrennte Plasmavolumen selbst nicht erhöht werden kann, so daß auch hierbei die Maximalmenge der zu eliminierenden Plasmabestandteile begrenzt ist.

Der Erfindung liegt die Aufgabe zugrunde, eine Vorrichtung der genannten Art zu schaffen, welche bei einfachem Aufbau und einfacher, betriebssicherer Anwendbarkeit eine selektive Elimination von Plasmabestandteilen unter Aufrechterhaltung eines hohen Wirkungsgrades gestatten.

Die Lösung der zugrundeliegenden Aufgabe erfolgt dadurch, daß vor dem Separationsfilter eine Verdünnungseinrichtung zur Verdünnung des zugeführten Blutes mit einer Verdünnungslösung angeordnet ist und daß stromab der Mischeinrichtung eine Entnahmeeinrichtung zur Abtrennung der Verdünnungslösung von dem Blut vorgesehen ist. Erfindungsgemäß ist somit sichergestellt, daß bereits vor Eintritt in den Separationsfilter das Blut des Patienten in ausreichender Weise verdünnt wird und daß gleichzeitig vermieden wird, daß die Verdünnungslösung in ihrer Gesamtheit dem Patienten rückgeführt wird. Die Verdünnungslösung dient somit lediglich dazu, entlang eines Teiles des Durchlaufweges des Blutes dieses in einem verdünnten Zustand zu halten.

Die erfindungsgemäße Vorrichtung ist in günstiger Weise so ausgebildet, daß die Entnahmeeinrichtung zur Separation der Verdünnungslösung von dem verdünnten Blut einen Hämofilter umfaßt.

Um eine Rückzirkulation der entnommenen Verdünnungslösung zu ermöglichen, ist die Entnahmeeinrichtung mittels einer Rückführleitung mit der Verdünnungseinrichtung verbunden.

Weiterhin kann im Bereich der Rückführleitung ein Speicher für die Verdünnungslösung vorgesehen sein, welcher beispielsweise in Form eines Beutels ausgebildet ist und dazu dient, Volumenveränderungen in dem System, beispielsweise beim Beginn der Behandlung auszugleichen.

Erfindungsgemäß kann die Abtrennvorrichtung, mittels welcher die zu entfernenden Plasmabestandteile von dem Blutplasma getrennt werden, in Form eines Adsorbers oder eines Kaskadenfilters ausgebildet sein. Dabei ist es weiterhin möglich, den Adsorber oder Kaskadenfilter rückzuspülen, um dessen Membranen zu reinigen.

Weiterhin umfaßt die erfindungsgemäße Vorrichtung bevorzugterweise eine Anzahl von Pumpen, nämlich eine Rückführpumpe in der Rückführleitung, eine Abtrennpumpe in der Zuleitung zur Abtrennvorrichtung, eine Fraktionierungspumpe zwischen der Mischungseinrichtung und der Entnahmeeinrichtung, eine arterielle Blutpumpe in der Blutzuführeinrichtung und eine venöse Blutpumpe in der Blutrückführeinrichtung. Durch Steuerung der Fördermengen der arteriellen Blutpumpe und der Rückführpumpe ist es möglich, das Maß der Verdünnung des zugeführten Blutes einzustellen. Im übrigen ist bevorzugterweise die Summe der Fördermengen der arteriellen Blutpumpe und der Rückführpumpe gleich der Fördermenge der Fraktionie rungspumpe, so daß eine gleichbleibende Volumenmenge dem System zu- bzw. abgeleitet wird.

Zur Steigerung der Filtrationsrate erweist es sich weiterhin als günstig, wenn eine Einrichtung für einen pulsierenden Fluß auf der Blutseite vor oder hinter dem Plasmaseparator oder Separationsfilter oder filtratseitig vor dem Franktionierungsmodul bzw. der Abtrennvorrichtung angeordnet ist.

Im folgenden wird die Erfindung anhand eines Ausführungsbeispiels in Verbindung mit der Zeichnung beschrieben. Dabei zeigt:

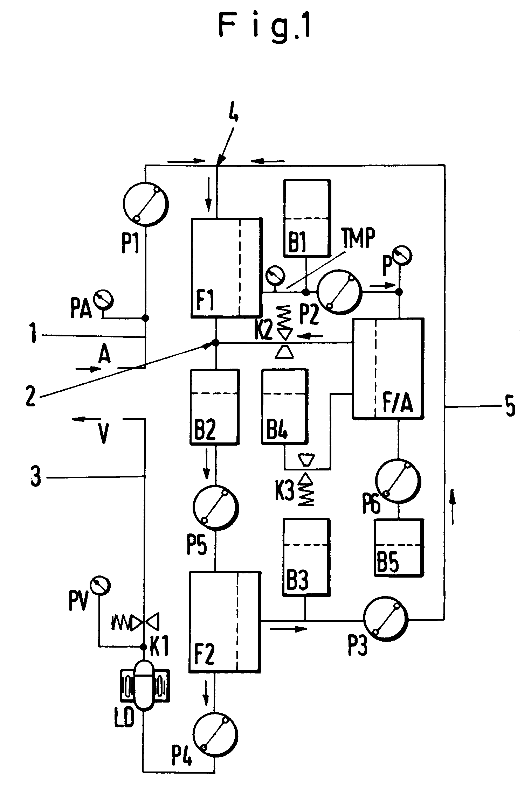

Fig. 1        eine schematische Darstellung eines Ausführungsbeispiels der erfindungsgemäßen Vorrichtung.

Die erfindungsgemäße Vorrichtung dient zur selektiven und spezifischen Elimination von Plasmabestandteilen aus Blut und wird nachfolgend am Beispiel einer Vorrichtung erläutert, mittels derer eine LDL-Reduktion möglich ist.

Der Anschluß der erfindungsgemäßen Vorrichtung erfolgt venovenös am Patienten, wobei verschiedene Anschlußmöglichkeiten gegeben sind. Es ist möglich, den Anschluß in üblicher 2--arm-Technik auszubilden, alternativ dazu ist jedoch ein Anschluß in single-needle Technik möglich, welcher dann in üblicher Weise eine Steuerung umfassen muß.

Die Blutseite der erfindungsgemäßen Vorrichtung umfaßt eine Blutzuführeinrichtung 1 sowie eine Blutrückführeinrichtung 3, welche in Form geeigneter Kanülen- oder Schlauchverbindungen ausgestaltet sind.

Die Blutzuführeinrichtung 1 umfaßt einen arteriellen Drucksensor PA sowie eine arterielle Blutpumpe P1. Das Blut wird einer Verdünnungseinrichtung 4 zugeführt, welche bei dem gezeigten Ausführungsbeispiel in Form eines T-Rohrstücks ausgebildet ist. Es ist jedoch auch möglich, die Verdünnungseinrichtung 4 in Form eines Mehrwegeventils, beispielsweise auch regelbar, auszubilden.

In der Verdünnungseinrichtung 4 wird das Blut mit einer Verdünnungslösung, beispielsweise einer Kochsalzlösung verdünnt.

Das verdünnte Blut wird einem Separationsfilter F1 (Plasmaseparator) zugeführt. Das korpuskuläre Blut verläßt den Separationsfilter 1 in Richtung auf eine Mischeinrichtung 2, während das Plasma über eine Abtrennpumpe P2 einer Abtrennvorrichtung F/A zugeleitet wird. In der Zuleitung des Blutplasmas ist zusätzlich ein TMP-Drucksensor sowie ein Fraktionierungsdrucksensor P vorgesehen. Weiterhin ist in der Leitung ein Plasmabeutel B1 angeordnet, welcher als Speicher dient.

In der Abtrennvorrichtung F/A werden die zu eliminierenden Plasmabestandteile aus dem Plasma abgetrennt.

Das aus der Abtrennvorrichtung F/A austretende Plasma wird unter Durchleitung durch eine Ruckspülklemme K2 der Mischeinrichtung 2 zugeführt, in welcher somit das Blutplasma mit den korpuskulären Bestandteilen des Blutes vermischt wird. Dieses Blut wird durch einen Beutel B2 geleitet, welcher somit sowohl die korpuskulären Blutbestandteile als auch das prozessierte Plasma umfaßt.

Nachfolgend an den Beutel B2 ist eine Fraktionierungspumpe P5 vorgesehen, mittels welcher das verdünnte Blut in eine Entnahmeeinrichtung F2 geleitet wird. In dieser Entnahmeeinrichtung F2 erfolgt eine Abtrennung oder Entnahme der Verdünnungslösung, welche über eine Rückführleitung der Verdünnungseinrichtung 4 zugeführt wird. In der Rückführleitung ist eine Pumpe P3 vorgesehen, weiterhin umfaßt die Rückführleitung einen Verdünnungslösungsbeutel B3.

Das nunmehr nicht mehr verdünnte Blut, welches die Entnahmeeinrichtung F2 verläßt, wird über eine Pumpe P4 (venöse Blutpumpe) der Blutrückführeinrichtung 3 zugeleitet. Diese Blutrückführeinrichtung 3 umfaßt weiterhin eine venöse Klemme K1, einen venösen Drucksensor PV sowie einen Luftdetektor LD.

Das Separationsfilter F1 kann beispielsweise ein von Fresenius vertriebener Plasmafilter Plasmaflux P2 sein, welcher in Form eines Membranfilters ausgebaut ist und welcher selbst bei der Elimination von Substanzen mit großem Molekulargewicht, z.B. Immunkomplexen mit 4 - 4 Mio. Dalton einen Siebkoeffizienten aufweist, welcher nahezu den Wert 1 erreicht. Die Entnahmeeinrichtung F2 (Hämofilter) kann beispielsweise in Form des Asahi-Hemofilters PAN-200 ausgebildet sein. Dieses kann beispielsweise ein Kapillarlumen von 200

μm und eine Kapillarwandstärke von 55 μm aufweisen. Die Abtrennvorrichtung FA kann entweder in Form eines großvolumigen Adsorbers oder eines Plasmakaskadenfilters vorliegen. Im Falle des Adsorbers können beispielsweise zwei in Reihe geschaltete Adsorber (vertrieben als Kaneka Liposorber LA 40) vorgesehen sein. Der Plasmakaskadenfilter kann in Form einer Asahi-Cascade AC 1760 ausgestaltet sein.

Die erfindungsgemäße Vorrichtung bietet somit die Möglichkeit, in der zweiten Behandlungsstufe soviel LDL zu extrahieren, wie sich in dem in der ersten Stufe separierten, verdünnten Plasmavolumen befindet. Insgesamt kann somit der Wirkungsgrad der Vorrichtung erheblich gesteigert werden.

Bei der Verwendung eines Kaskadenfilters als Abtrennvorrichtung F/A erfolgt im Verlauf der Behandlung mindestens eine Rückspülung mit isotonischer Kochsalzlösung. Zur Rückspülung wird die Klemme K2 geschlossen, weiterhin wird die Pumpe P2 angehalten, so daß der diesbezügliche Leitungsweg unterbrochen ist. Weiterhin wird die Klemme K3 geöffnet, welche eine Verbindung mit einem Beutel B4 ermöglicht, in welchem sich die isotone Kochsalzlösung befindet. Diese wird durch die Pumpe P6 durch den Filter bzw. die Abtrennvorrichtung gepumpt und in dem Beutel B5 aufgefangen.

Die Steuerung der zweiten Stufe, in welcher das Blutplasma behandelt wird, erfolgt unter Zuhilfenahme des durch den Drucksensor P ermittelten Druckes vor der Abtrennvorrichtung F/A. Vorteilhafterweise ist dabei eine rechnergesteuerte Verfahrensführung mit automatischem Spülzyklus bei Verwendung eines Kaskadenfilters vorgesehen.

Das Maß der Verdünnung des Blutes wird mittels der arteriellen Blutpumpe P1 und der Rückführpumpe (Dilutionspumpe) P3 eingestellt. Der Fluß der venösen Blutpumpe P4 und der arteriellen Blutpumpe P1 sind im Normalbetrieb gleich groß, um dem Patienten die entnommenen Volumina rückführen zu können. Zur Aufkonzentration des Blutes ist der Fluß der venösen Blutpumpe P4 kleiner als der der arteriellen Blutpumpe P1. Die Summe der Fördermengen der arteriellen Blutpumpe P1 und der Dilutionspumpe P3 ist gleich der Fördermenge der Fraktionierungspumpe P5.

Im folgenden wird die Funktionsweise der erfindungsgemäßen Anordnung beschrieben. Nach Anschluß der Katheder an den Blutkreislauf des Patienten wird mittels der Pumpe P1 das durch Blutzuführeinrichtung 1 geförderte Blut der Verdünnungseinrichtung 4 zugeführt. Die Verdünnungseinrichtung 4 wird zu Beginn des gesamten Verfahrensablaufes aus dem Speicher B3 durch Betätigung der Pumpe P3 mit Verdünnungsflüssigkeit versorgt. Es erfolgt somit eine Verdünnung des zugeführten Blutes, welches in der entsprechend verdünnten Form dem Separationsfilter F1 zugeführt wird. In dem Separationsfilter F1 wird das Blutplasma abgetrennt und mittels der Pumpe P2 der Abtrennvorrichtung F/A zugeführt. Aus dieser verläßt das Blutplasma durch eine im einzelnen nicht bezeichnete Schlauchleitung unter passieren der Klemme K2 den Plasmakreislauf und wird an der Mischeinrichtung 2 mit dem korpuskulären Blut vermischt. Dieses gelangt durch einen Zwischenspeicher B2 und wird mittels der Pumpe P5 der Entnahmeeinrichtung F2 zugeführt. In dieser erfolgt eine Abtrennung der Verdünnungsflüssigkeit, welche über die Pumpe P3 in die Rückführleitung 5 gefördert wird. Der Speicher B3 dient sowohl als Speicher zum Ausgleich von Flüssigkeitsschwankungen während des Betriebs, als auch, wie bereits erwähnt, während der Anlaufphase. Zusätzlich wird der Speicher B3 dann verwendet, wenn der Blutbehandlungsvorgang beendet werden soll, wobei dann die Rückführpumpe P3 einen Rückfluß der Verdünnungsflüssigkeit verhindert. Das nach Durchlauf durch die Entnahmeeinrichtung F2 wieder auf die ursprüngliche Konzentration gebrachte Blut wird über die venöse Blutpumpe P4 dem menschlichen Körper zugeführt. Um eine unerwünschte Rückströmung bei Beendigung des Behandlungsvorganges und/oder während einer Rückspülung der Abtrennvorrichtung F/A zu verhindern, ist in der Blutrückführleitung 3 zusätzlich eine Klemme K1 vorgesehen.

In einem Laboraufbau mit 0,5 l Vollblut und einem Adsorber als Fraktionierungsmodul F/A wurden die folgenden Werte für die Fördermengen der einzelnen Pumpen verwendet:

| arterielle Blutpumpe P1 | 30 ml/min |
|---|---|
| venöse Blutpumpe P4 | 30 ml/min |
| Plasmapumpe P2 | 100 ml/min |
| Dilutionspumpe P3 | 160 ml/min |
| Fraktionierungspumpe P5 | 190 ml/min. |

Das Volumen der Beutel war dabei wie folgt:

| Plasmabeutel B1 | kleiner 500 ml |
|---|---|
| fraktioniertes Plasma und Blut im Beutel B2 | kleiner 500 ml |
| Verdünnungslösung im Beutel B3 | kleiner 2000 ml. |

Das extrakorporale Blutvolumen betrug etwa 300 ml.

Die blutseitige Verdünnung mit anschließender blutseitiger Aufkonzentration weist die folgenden Vorteile auf: es erfolgt eine Erhöhung der Plasmaausbeute des Primär-Plasmaseparators. Weiterhin erfolgt eine Erhöhung der Effektivität des Sekundär-Moduls, wobei in diesem Modul kein Channeling auftritt. Weiterhin ist wahlweise ein Einsatz eines Adsorbers oder Filters als Sekundär-Modul in der gleichen Einrichtung möglich. Die Abtrennung erfolgt substituatfrei. Weiterhin erfolgt keine Desorption des Adsorbers mit toxischer Lösung.

Die Erfindung ist nicht auf das gezeigte Ausführungsbeispiel beschränkt, vielmehr ergeben sich für den Fachmann im Rahmen der Erfindung weitere Abwandlungs- und Modifikationsmöglichkeiten.

**Patentansprüche**

1. Vorrichtung zur selektiven Elimination von Plasmabestandteilen aus Blut mit einer Blutzuführeinrichtung (1), einem Separationsfilter (F1) zur Abtrennung von Plasma von dem Blut, einer Abtrennvorrichtung (F/A) zur Entfernung der jeweiligen Plasmabestandteile, einer Mischeinrichtung (2) zum Mischen des prozessierten Plasmas mit den korpuskularen Blutbestandteilen des Blutes und einer Blutrückführeinrichtung (3), dadurch gekennzeichnet, daß vor dem Separtionsfilter (F1) eine Verdünnungseinrichtung (4) zur Verdünnung des zugeführten Blutes mit einer Verdünnungslösung angeordnet ist und daß stromab der Mischeinrichtung (2) eine Entnahmeeinrichtung (F2) zur Abtrennung der Verdünnungslösung von dem Blut vorgesehen ist.

2. Vorrichtung nach Anspruch 1, dadurch gekennzeichnet, daß die Entnahmeeinrichtung (F2) einen Hämofilter umfaßt.

3. Vorrichtung nach Anspruch 1 oder 2, dadurch gekennzeichnet, daß die Entnahmeeinrichtung (F2) mittels einer Rückführleitung (5) mit der Verdünnungseinrichtung (4) verbunden ist, wobei im Bereich der Rückführleitung (5) vorzugsweise ein Speicher (B3) für die Verdünnungslösung vorgesehen ist und in der Rückführleitung (5) eine Rückführpumpe (P3) zwischengeschaltet ist.

4. Vorrichtung nach einem der Ansprüche 1 - 3, dadurch gekennzeichnet, daß die Abtrennvorrichtung (FA) einen Adsorber oder einen Plasmakaskadefilter umfaßt.

5. Vorrichtung nach einem der Ansprüche 1 - 4, dadurch gekennzeichent, daß in der Zuleitung zur Abtrennvorrichtung (F/A) eine Abtrennpumpe (P2) angeordnet ist.

6. Vorrichtung nach einem der Ansprüche 1 - 5, dadurch gekennzeichnet, daß zwischen der Mischeinrichtung (2) und der Entnahmeeinrichtung (F2) eine Fraktionierungspumpe (P5) zwischengeschaltet ist.

7. Vorrichtung nach einem der Ansprüche 1 - 6, dadurch gekennzeichnet, daß in der Blutzuführeinrichtung (1) eine arterielle Blutpumpe (P1) und in der Blutrückführeinrichtung (3) eine venöse Blutpumpe (P4) angeordnet sind, wobei das Maß der Verdünnung des zugeführten Blutes mittels der arteriellen Blutpume (P1) und der Rückführpumpe (P3) einstellbar ist und vorzugsweise die Summe der Fördermengen der arteriellen Blutpumpe (P1) und der Rückführpumpe (P3) gleich der Fördermenge der Fraktionierungspumpe (P5) ist.

8. Vorrichtung nach einem der Ansprüche 1 - 7, dadurch gekennzeichnet, daß die Abtrennvorrichtung (F/A) rückspülbar ausgebildet ist.

9. Vorrichtung nach einem der Ansprüche 1 - 8, dadurch gekennzeichnet, daß vor der Abtrennvorrichtung (F/A) ein Drucksensor (P) angeordnet ist.

10. Vorrichtung nach einem der Ansprüche 1 - 9, dadurch gekennzeichnet, daß in der Zuleitung oder der Ableitung des Separtionsfilters (F1) vor der Abtrennvorrichtung (F/A) eine Einrichtung für einen pulsierenden Blutfluß angeordnet ist.

**Claims**

1. An apparatus for the selective elimination of plasma components from blood comprising a blood feed device (1), a separation filter (F1) for the separation of plasma from the blood, a segregating device (F/A) for the removal of the respective plasma components, a mixing device (2) for mixing the processed plasma with the corpuscular blood components and a blood return device (3), characterized in that a diluting device (4) for dilution of the fed blood with a diluting solution is placed upstream from the separation filter (F1) and that a removal device (F2) is placed downstream from the mixing device (2) for segregating the diluting solution from the blood.

2. The apparatus as claimed in claim 1, characterized in that said removal device (F2) includes a hemofilter.

3. The apparatus as claimed in claim 1 or 2, characterized in that said removal device (F2) is connected via a return line (5) with said diluting device (4) whereby within the area of said return line (5) preferably a storage means (B3) for the diluting solution is provided and a return pump (P3) is interconnected in said return line (5).

4. The apparatus as claimed in any one of claims 1 through 3, characterized in that the segregating device (F/A) includes an adsorber or a plasma cascade filter.

5. The apparatus as claimed in any of claims 1 through 4, characterized in that a segregating pump (P2) is arranged on the feed line leading to the segregating device (F/A).

6. The apparatus as claimed in any one of claims 1 through 5, characterized in that a fractionating pump (P5) is interconnected between the mixing device (2) and the removal device (F2).

7. The apparatus as claimed in any one of claims 1 through 6, characterized in that an arterial blood pump (P1) is placed in the blood feed device (1) and a venous blood pump (P4) is placed in the blood return device (3) with the degree of dilution of the blood fed by said arterial blood pump (P1) being adjustable and that preferably the sum of the displacements of the arterial blood pump (P1) and of the return pump (P3) is equal to the displacement of the fractionating pump (5).

8. The apparatus as claimed in any one of claims 1 through 7, characterized in that the segregating device (F/A) is adapted for the passage of a reverse flushing flow therethrough.

9. The apparatus as claimed in any one of claims 1 through 8, characterized by a pressure sensor (P) placed upstream from the segregating device (F/A).

10. The apparatus as claimed in any one of claims 1 through 9, characterized in that a device is placed on the feed line or the output line of the separation filter (F1) upstream from the segregating device (F/A) for producing a pulsating blood flow.

## Revendications

1. Dispositif d'élimination sélective de composants du plasma du sang, comportant un dispositif (1) d'amenée du sang, un filtre de séparation (F1) pour séparer le plasma du sang, un dispositif de séparation (F/A) pour éliminer les composants respectifs du plasma, un dispositif de mélange (2) pour mélanger le plasma traité au composants corpusculaires du sang et un dispositif (3) de renvoi du sang, caractérisé en ce qu'en amont du filtre de séparation (F1) est disposé un dispositif de dilution (4) servant à diluer le sang amené avec une solution de dilution, et qu'en aval du dispositif de mesure (2) il est prévu un dispositif de prélèvement (F2) servant à séparer la solution de dilution, du sang.

2. Dispositif selon la revendication 1, caractérisé en ce que le dispositif de prélèvement (F2) est un filtre hémofiltration.

3. Dispositif selon la revendication 1 ou 2, caractérisé en ce que le dispositif de prélèvement (F2) est raccordé, au moyen d'une canalisation de renvoi (5) au dispositif de dilution, et de préférence un accumulateur (B3) pour la solution de dilution est prévu au niveau de la canalisation de renvoi (5) et une pompe de renvoi (B3) est intercalée dans la canalisation de renvoi (5).

4. Dispositif selon l'une des revendications 1-3, caractérisé en ce que le dispositif de séparation (FA) comprend un adsorbeur ou un filtre cascade à plasma.

5. Dispositif selon l'une des revendications 1 - 4, caractérisé en ce qu une pompe de séparation (P2) est montée dans la canalisation d'amenée aboutissant au dispositif de séparation (F/A).

6. Dispositif selon l'une des revendications 1 - 5, caractérisé en ce qu'une pompe de fractionnement (P5) est intercalée entre le dispositif de mélange (2) et le dispositif de prélèvement (F2).

7. Dispositif selon l'une des revendications 1 - 6, caractérisé en ce qu'une pompe pour le sang artériel (P1) est montée dans le dispositif (1) d'amenée du sang et qu'une pompe (P4) pour le sang veineux est montée dans le dispositif (3) de renvoi du sang, et que le degré de dilution du sang amené est réglable au moyen de la pompe (P1) pour le sang artériel et de la pompe de renvoi (P3) et de préférence, la somme des débits de la pompe (P1) pour le sang artériel et de la pompe de renvoi (P3) est égale au débit de la pompe de fractionnement (P5).

8. Dispositif selon l'une des revendications 1 - 7, caractérisé en ce que le dispositif de séparation (F/A) est agencé de manière à être soumis à un lavage à contre-courant.

9. Dispositif selon l'une des revendications 1 - 8, caractérisé en ce qu'un capteur de pression (P) est disposé en amont du dispositif de séparation (F/A).

10. Dispositif selon l'une des revendications 1 - 9, caractérisé en ce qu'un dispositif servant à délivrer un flux sanguin pulsatoire est disposé dans la canalisation d'amenée ou la canalisation d'évacuation du filtre de séparation (F1) en amont du dispositif de séparation (F/A).

# F i g.1